# EUROPÄISCHE PATENTSCHRIFT

(11) **EP 3 446 108 B1**
(45) Veröffentlichungstag und Bekanntmachung des Hinweises auf die Patenterteilung: **24.07.2019**
(21) Anmeldenummer: 18709931.2
(22) Anmeldetag: 09.02.2018
(51) Int. Cl.: G01N 21/75, G01N 21/85, G01N 15/02, C12M 1/02

(54) **VERFAHREN UND VORRICHTUNG ZUR ABSTIMMUNG OPTISCHER MESSUNGEN AN KONTINUIERLICH DURCHMISCHTEN REAKTOREN**
METHOD AND DEVICE FOR TUNING OPTICAL MEASUREMENTS ON CONTINUOUSLY MIXED REACTORS
PROCÉDÉ ET DISPOSITIF DE COORDINATION DE MESURES OPTIQUES DANS DES RÉACTEURS CONTINUELLEMENT MÉLANGÉS

(30) Priorität: 17.02.2017 DE 102017001588
(43) Veröffentlichungstag der Anmeldung: 27.02.2019
(73) Patentinhaber: Aquila Biolabs GmbH, 52499 Baesweiler (DE)
(72) Erfinder: HERZOG, Konrad, 52499 Baesweiler (DE); FRANK, David, 52499 Baesweiler (DE)
(74) Vertreter: Roth, Andy Stefan
(86) Internationale Anmeldenummer: PCT/EP2018/053239
(87) Internationale Veröffentlichungsnummer: WO 2018/149739

(56) Entgegenhaltungen:
- DE-A1-102011 000 891
- DE-B3-102014 001 284
- FR-A1- 2 867 278
- US-A1- 2005 019 900
- US-A1- 2008 312 843
- US-A1- 2013 033 272
- JÖRG SCHMIDT-HAGER ET AL: "Noninvasive online biomass detector system for cultivation in shake flasks", ENGINEERING IN LIFE SCIENCES, Bd. 14, Nr. 5, 24. Juli 2014 (2014-07-24), Seiten 467-476, XP055194928, ISSN: 1618-0240, DOI: 10.1002/elsc.201400026
- PATEL C ET AL: "Combined spectrophotometric-electrochemical impedance imaging system for biofilm research", JOURNAL OF THE ASSOCIATION FOR LABORATORY AUTOMATION, ELSEVIER, Bd. 10, Nr. 1, Februar 2005 (2005-02), Seiten 16-23, XP027677788, ISSN: 1535-5535 [gefunden am 2005-02-01]

## Beschreibung

### Technisches Gebiet

Die Erfindung betrifft ein Verfahren und eine Vorrichtung zur Abstimmung optischer Messungen an kontinuierlich durchmischten Reaktoren. Sie ist insbesondere anwendbar zur Steigerung der Robustheit und Vertrauenswürdigkeit von zur Prozessüberwachung der Kultivierung von Zellen sowie chemischer Reaktionen eingesetzten optischen Messungen an kontinuierlich durchmischten Reaktoren, insbesondere Schüttelkolben, Mikrotiterplatten, Rührkesselfermentern und Reaktionsröhrchen.

Kontinuierlich durchmischte Reaktoren kommen in vielen Bereichen der chemischen, biologischen und biotechnologischen Industrie und Forschung zum Einsatz, insbesondere zur Durchführung chemischer Reaktionen und Synthesen sowie zur Kultivierung lebender Zellen. Die Überwachung dieser Prozesse erfolgt häufig mittels optischer Verfahren, wie beispielsweise Streulichtmessungen, Absorptionsmessungen oder Fluoreszenzmessungen, welche sich üblicherweise durch die Kombination mindestens einer Lichtquelle und mindestens eines Lichtsensors auszeichnen. Robuste optische Messverfahren erfordern reproduzierbare Messbedingungen, insbesondere bezüglich der sich im optischen Pfad befindlichen Probe. Jede Änderung dieser Messbedingung und jede Modifikation des optischen Pfades zwischen Lichtquelle und Lichtsensor beeinflusst nachteilig die Qualität und Vertrauenswürdigkeit der optischen Messung.

Kontinuierlich durchmischte Reaktoren zeichnen sich durch bewegte Komponenten aus, welche die Durchführung robuster und vertrauenswürdiger Messungen erschweren durch die Behinderung der Einstellung reproduzierbarer Messbedingungen durch einen stabilen und vergleichbaren optischen Pfad. In geschüttelten Reaktoren, insbesondere in Schüttelkolben, Schüttelflaschen, Fermentationssäcken, Reaktionsröhrchen und Mikrotiterplatten verändert sich kontinuierlich die Form und Verteilung des Reaktorinhalts und erschwert dadurch optische Messungen. In gerührten Reaktoren sind Form und Verteilung des Reaktorinhalts zwar zumeist konstant, jedoch befinden sich in ihnen sich bewegende Rührer, welche ebenfalls mit optischen Messungen interferieren können und diese somit erschweren.

### Stand der Technik

Aus dem Stand der Technik sind Verfahren und Vorrichtungen bekannt, welche die Durchführbarkeit optischer Messungen an kontinuierlich durchmischten Reaktoren verbessern, indem sie auf unterschiedliche Weise versuchen, reproduzierbare Messbedingungen zu erzeugen oder die Veränderungen des optischen Pfades in die Auswertung der Messsignale einzubeziehen.

DE102004017039A1 offenbart Verfahren und Vorrichtung zur Erfassung von Prozessparametern in mehreren geschüttelten Mikroreaktoren. Als Maßnahmen zur Abstimmung der optischen Messung wird zunächst die Verwendung einer Blitzlampe als Lichtquelle genannt, deren Lichtblitze auf die Rotationsbewegung des Schüttlers oder auf einen Positionsgeber (Beschleunigungssensor, Lichtschranke oder Hall-Sensor) abgestimmt sind, um zu verhindern, dass optische Messungen in solchen Momenten durchgeführt werden, in denen sich keine Flüssigkeit über dem Sensor und der Lichtquelle befindet. Weiterhin wird eine Ausführungsform offenbart, in der durch eine hohe Blitz- und Messfrequenz, welche über der Schüttelfrequenz des Schüttlers liegt, viele Messpunkte aufgenommen werden, um eine Schwebung des Messsignals zu verhindern.

DE102014001284B3 offenbart Verfahren, Vorrichtung und System zur automatisierten Bestimmung optischer Dichten oder der Veränderung optischer Dichten von Reaktionsgemischen in geschüttelten Reaktoren. Hier wird keine Abstimmung der optischen Messung vorgenommen, sondern es werden mit hoher Frequenz kontinuierliche optische Messungen durchgeführt, wobei die Schwankungen des optischen Signals unter Annahme einer konstanten Messgröße als Schwankungen der Form und Verteilung der Flüssigkeit im Reaktor interpretiert werden, sodass mittels dieser Informationen eine angepasste mathematische Auswertung und Korrektur der optischen Signale erfolgen kann.

DE102011000891A1 offenbart Verfahren und Vorrichtung zum Bestimmen einer Veränderlichen einer sich in einem bewegten Behältnis befindlichen Probe. Beschrieben wird hier eine Abstimmung der optischen Messung auf den Bewegungszustand der Probe, um zu verhindern, dass optische Messungen in solchen Momenten durchgeführt werden, in denen sich keine oder zu wenig Flüssigkeit über dem Sensor und der Lichtquelle befindet. Dazu wird ein Synchronisierungsmittel offenbart, welches die definierten Bewegungen des Reaktors erfasst durch Messungen der Beschleunigung oder Position oder durch Erfassung der Motorbewegung während des Schüttelns. Weiterhin wird offenbart, dass zum Zweck der Bestimmung des aktuellen Flüssigkeitszustands weitere Parameter wie Viskosität, Schüttelfrequenz, Schüttelradius, Reaktorgeometrie und Füllvolumen herangezogen werden können. Zudem ist eine Ausführungsform offenbart, in der zur Abstimmung der Bewegungszustand der Probe direkt über eine zusätzliche optische Messung erfasst wird.

Die durch den Stand der Technik offenbarten Verfahren und Vorrichtungen, welche die Durchführbarkeit optischer Messungen an kontinuierlich durchmischten Reaktoren verbessern sollen, weisen nachteilige Limitationen ihrer Anwendbarkeit auf, welche im Folgenden dargestellt werden.

Eine Abstimmung optischer Messungen auf den Bewegungszustand des Reaktors, wie durch Beschleunigungs- oder Positionssensoren sowie durch die Kopplung an Motorparameter vernachlässigt nachteilig, dass infolge der Trägheit des Reaktorinhalts dieser insbesondere in geschüttelten Systemen immer der Bewegung des Reaktors nacheilt. Zwar wird, wie in DE102011000891A1 offenbart, durch Heranziehung weiterer Parameter wie Viskosität, Schüttelfrequenz, Schüttelradius, Reaktorgeometrie und Füllvolumen die Berechnung einer zeitlichen Verschiebung der Relativbewegung zwischen Reaktor und Reaktorinhalt möglich, jedoch sind derartige Berechnungen anfällig gegenüber jeglicher Änderung dieser Parameter, sodass daraus signifikante Fehlbestimmungen des Bewegungszustandes des Reaktorinhalts folgen und somit die optische Messung falsch abgestimmt wird. Von herausragender Bedeutung für die Form, Verteilung und den Bewegungszustand des Reaktorinhalts sind seine Viskosität, seine Dichte und die Stärke der Reibungskräfte zwischen Reaktorinhalt und Reaktorwand. Insbesondere bei der Kultivierung von Zellen können sich diese Parameter jedoch signifikant ändern (z.B. Viskosität durch filamentöses Wachstum oder durch Ausscheidung von Biopolymeren), sodass weder rohe Beschleunigungs- oder Positionssensorendaten noch von ihnen durch bekannte Parameter abgeleitete Daten zur Beurteilung der Form, Verteilung und des Bewegungszustandes des Reaktorinhalts geeignet sind. Somit kann auch keine korrekte Abstimmung optischer Messungen erfolgen.

Die Erfassung der Form, Verteilung und des Bewegungszustandes des Reaktorinhalts im laufenden Schüttelbetrieb durch optische Messungen ist zwar grundsätzlich möglich, allerdings sind ebendiese optischen Messverfahren sehr anfällig gegenüber äußeren, sich häufig prozessbegleitend ändernden Einflussfaktoren, welche letztendlich zu einer Fehlbeurteilung der Form, Verteilung und des Bewegungszustandes des Reaktorinhalts führen, sodass keine korrekte Abstimmung der eigentlichen optischen Messungen mehr erfolgen kann. Solche nachteiligen Einflussfaktoren sind unter anderem interferierendes Außenlicht, Variationen in der Positionierung des Reaktors über dem optischen Sensor, unterschiedliche Reaktormaterialien und optische Inhomogenitäten dieser Materialien, sich ändernde optische Eigenschaften des Reaktorinhalts, etc. Die Vielzahl der äußeren Einflussfaktoren einer optischen Erfassung der Form, Verteilung und des Bewegungszustandes des Reaktorinhalts führen dazu, dass in vielen Fällen nicht mehr unterschieden werden kann, ob eine Änderung des optischen Signals solcher Vorrichtungen durch eine Änderung der Form, Verteilung oder des Bewegungszustandes des Reaktorinhalts hervorgerufen wurde, oder aber ob die Signaländerung eine Folge sich ändernder oder anderweitig nicht konstanter innerer oder äußerer Einflussfaktoren ist.

Sämtliche aus dem Stand der Technik offenbarten Verfahren und Vorrichtungen weisen infolge ihrer Abhängigkeit von definierten Rahmenbedingungen (z.B. Schüttelfrequenz, Füllvolumen, Viskosität, laminares Strömungsverhalten) weitere Nachteile auf, die ihre Einsetzbarkeit zur Abstimmung optischer Messungen an kontinuierlich durchmischten Reaktoren nachteilig begrenzen.

Die häufig zur besseren Durchmischung in Reaktoren eingesetzten Schikanen und Strömungshindernisse (z.B. Schikanekolben, "Flowerplates", Prallbleche) führen durch Erzeugung turbulenter Strömungen zu signifikanten und nur äußerst schwierig in Echtzeit zu berechnenden Abweichungen von den für die Berechnung der zeitlichen Verschiebung zwischen Reaktorbewegung und der Bewegung des Reaktorinhalts notwendigen fluiddynamischen Randbedingungen. Dadurch wird die Genauigkeit der Abstimmung optischer Messungen an kontinuierlich durchmischten Reaktoren nachteilig herabgesetzt. Zudem führen derartige Strömungshindernisse häufig zu starker Blasen- oder Schaumbildung, welche optische Messungen der Form, Verteilung und des Bewegungszustandes des Reaktorinhalts, insbesondere bei niedrigen Füllvolumina, unmöglich macht oder zumindest signifikant erschwert.

Auch eine Änderung des Volumens des Reaktorinhalts, insbesondere durch Probenentnahme oder Zugabe von Substanzen (z.B. Fed-Batch-Fermentation, pH-Regelung, Antischaumzugabe, Zugabe von Proteinexpressionsinduktoren), beeinflusst die aus dem Stand der Technik offenbarten Verfahren und Vorrichtungen nachteilig bezüglich der Genauigkeit der zeitlichen Abstimmung optischer Messungen an kontinuierlich durchmischten Reaktoren. Ursächlich dafür ist, dass Volumenänderungen immer zu Änderungen der fluidmechanischen Bedingungen im Reaktor führen, welche durch Beschleunigungs- und Positionssensoren nicht erfasst werden können und welche durch optische Verfahren aufgrund ihrer Störanfälligkeit gegenüber anderen Außenparametern nur begrenzt aufgelöst werden können.

Es ist daher Aufgabe der vorliegenden Erfindung, ein verbesserter Verfahren zur Abstimmung optischer Messungen an kontinuierlich durchmischten Reaktorenanzugeben, insbesondere mittels dessen die Abstimmung optischer Messungen an kontinuierlich durchmischten Reaktoren robust und unabhängig von sich prozessbegleitend ändernden fluidischen oder optischen Parametern des Reaktorinhalts ermöglicht wird. Insbesondere ist dabei ein Verfahren anzugeben, um mindestens eine optische Messung auf die Form, Verteilung oder den Bewegungszustand des Reaktorinhalts abzustimmen. Die der Erfindung zugrundeliegende Aufgabe wird gelöst durch ein Verfahren nach Anspruch 1 sowie eine Vorrichtung nach Anspruch 8; bevorzugte Ausgestaltungen ergeben sich aus den Unteransprüche sowie der Beschreibung.

### Definitionen

Zur Sicherstellung der Klarheit einiger in der Beschreibung verwendeter Begriffe, werden diese nachfolgend und im Verlauf der Beschreibung definiert und erläutert.

Ein Reaktor ist insbesondere ein Behältnis, welches insbesondere zur Kultivierung von Organismen oder zur Durchführung chemischer und biochemischer Reaktionsprozesse eingesetzt werden kann. Weitere Einsatzbereiche von Reaktoren sind unter anderem biokatalytische Prozesse unter Verwendung von Organismen und/oder Biomolekülen sowie andere chemische und/oder physikalische Prozesse, wobei vom Begriff Prozess alle Arten der Umwandlung, Auftrennung, Zusammenführung, Durchmischung, Größenänderung von insbesondere chemischen Stoffen, Organismen, Partikeln, Lösungen, Emulsionen und Schäumen umfasst sind. Reaktoren im Sinne der Erfindung umfassen insbesondere Rührkesselfermenter, Blasensäulenfermenter, Schüttelkolben, T-Flasks, Mikrotiterplatten, Deep-Well-Plates, Schüttelfässer, Fermentation-Bags, Mehrzweckröhrchen und Zellkulturschalen. Reaktoren können gegenüber ihrer Umwelt geschlossen oder offen sein.

Der Reaktorinhalt umfasst sämtliche Materie, welche sich innerhalb der Außenhülle des Reaktors befindet. Der Reaktorinhalt umfasst eine oder mehrere Phasen und/oder setzt sich aus einer oder mehreren Phasen zusammen.

Die Durchmischung eines Reaktors beziehungsweise des Reaktorinhalts bezeichnet insbesondere jedes Verfahren, den Reaktorinhalt dergestalt zu beeinflussen, dass sich mindestens zwei, zu unterschiedlichen Zeiten aufgenommene Zustände der Verteilung des Reaktorinhalts und seiner Bestandteile im Reaktor, nicht gleichen. Übliche Durchmischungsverfahren nutzen dazu insbesondere mechanische oder thermische/thermodynamische Verfahren, insbesondere aber nicht ausschließlich Schüttelverfahren, Rührverfahren und Diffusionsverfahren. Die Durchmischungsfrequenz ist dabei für periodisch wiederholte Durchmischungsverfahren (Orbitalschütteln, Wippschütteln, Rühren) die Anzahl der Durchmischungsperioden pro Zeit.

Die Koexistenz nicht vollständig mischbarer Stoffsysteme in einem Reaktorinhalt führt zur Bildung von Phasen. Eine Phase ist dabei ein Stoffsystem, welches sich durch einen dominierenden Aggregatszustand auszeichnet und welches nicht vollständig mit anderen Phasen mischbar ist. Typische Beispiele hierfür sind Gasphasen, Flüssigphasen und Festphasen. Der Begriff Phase wird im Sinne der Erfindung jedoch nicht nur für reine Aggregatszustände verwendet, sondern auch für solche Stoffsysteme, die sich durch ein oder mehrere mindestens annährend homogen mischbare, suspendierbare oder emulgierbare Stoffsysteme oder Stoffe auszeichnen. Beispiele für reine Phasen sind flüssiges Wasser oder gasförmiger Stickstoff. Beispiele für Phasen im erweiterten Definitionsbereich der Erfindung nach oben beschriebenen Kriterien sind Luft (gasförmiges homogenes Gemisch), Kulturmedium mit Zellen (nahezu homogene Suspension von Zellen in einer homogenen wässrigen Lösung von Medienbestandteilen wie Salzen, Proteinen, Kohlenhydraten, etc.) oder eine Rührturbine aus Edelstahl (nahezu homogene Mischung von Eisen und Legierungselementen). Homogenität ist dabei skalenabhängig von der Auflösung der eingesetzten Permittivitätssensoren, sodass als homogene Phase gilt, was der eingesetzte Permittivitätssensor nicht mehr bezüglich lokaler Permittivitätsänderungen als inhomogen erfassen kann. Infolge ihrer Homogenität kann jeder Phase eine phasenspezifische Permittivität zugeordnet werden, welche sich als volumetrisch und molar gewichteter Mittelwert über die relativen Permittivitäten aller Komponenten der Phase ergibt. Als Beispiel sei hier genannt die Phase "Kulturmedium mit Zellen", deren mittlere Permittivität sich frequenzabhängig als gewichteter Mittelwert der relativen Permittivitäten der Hauptkomponente Wasser sowie aller weiteren Komponenten ergibt (Ionen, Zellen, gelöste Moleküle, gelöste Gase, etc.). Eine weitere bespielhafte Phase ist Luft, deren mittlere Permittivität sich frequenzabhängig als gewichteter Mittelwert der relativen Permittivitäten ihrer Komponenten, insbesondere Stickstoff, Sauerstoff, Wasserdampf, Kohlendioxid, Edelgase, etc. ergibt.

Die Kombination phasenspezifischer Permittivitäten mit der durchmischungsbedingten Veränderung der Form, Verteilung oder des Bewegungszustandes mindestens einer Phase des Reaktorinhalts resultiert in lokalen Permittivitäten und Permittivitätsunterschieden. So unterscheidet sich die lokale Permittivität an einem Ort im Reaktor, an dem Wasser ist, signifikant von der lokalen Permittivität an einem Ort, an dem Luft ist. Infolgedessen erlaubt die Erfassung lokaler Permittivitäten und lokaler Permittivitätsänderungen eine Beurteilung und/oder quantitative Erfassung der Form, Verteilung oder des Bewegungszustandes des Reaktorinhalts oder mindestens einer Phase des Reaktorinhalts.

Permittivitätssensoren erfassen mindestens eine lokale Permittivität. Dies erfolgt insbesondere über mindestens ein elektrisches Feld, welches sowohl mit mindestens einem Permittivitätssensor als auch mit dem Reaktorinhalt am zu untersuchenden Ort interagiert. Das Volumen des untersuchten Ortes hängt insbesondere, aber nicht ausschließlich ab von der Stärke, Form, Polarität und Frequenz des elektrischen Feldes sowie von der Zusammensetzung und Phasenverteilung des Reaktorinhalts im Interaktionsbereich des elektrischen Feldes. Permittivitätssensoren können insbesondere als Kapazitätssensoren mit einer oder mehreren Elektroden implementiert werden. Das üblicherweise für die Erfassung von Permittivitäten erforderliche elektrische Feld kann entweder über die sensorische Elektrode selbst, oder durch mit der sensorischen Elektrode interagierende zusätzliche Elektroden erzeugt werden. Die Positionierung von Permittivitätssensoren in einem bekannten Abstand zu mindestens einer abzustimmenden optischen Messanordnung erlaubt eine Erfassung der lokalen Permittivität mit räumlichem Bezug zur optischen Messposition, sodass daraus resultierend eine Abstimmung der optischen Messung auf Form, Verteilung oder Bewegungszustand des Reaktorinhalts oder mindestens einer Phase des Reaktorinhalts relativ zur optischen Messanordnung erfolgen kann.

Die Abstimmung einer optischen Messung auf die Form, Verteilung oder den Bewegungszustand des Reaktorinhalts umfasst alle Verfahren, welche die Planung, Durchführung, Verarbeitung, Auswertung oder Visualisierung der optischen Messung in Zusammenhang mit der Form, Verteilung oder dem Bewegungszustand des Reaktorinhalts bringen. Abstimmungen optischer Messungen im Sinne der Erfindung sind insbesondere, aber nicht ausschließlich, die Synchronisation der optischen Messung auf mindestens einen Zustand der Form, Verteilung oder des Bewegungszustands des Reaktorinhalts sowie die Einbeziehung von Informationen zur Form, Verteilung oder zum Bewegungszustand des Reaktorinhalts in die Auswertung der optischen Messdaten (beispielsweise durch Korrektur oder Normierung). Zum Zweck der Abstimmung können Randbedingungen oder Grenzwerte bezüglich erfasster Permittivitätssignale oder aus ihnen abgeleiteter Werte zum Einsatz kommen.

Verfahren zur Abstimmung einer optischen Messung auf die Form, Verteilung oder den Bewegungszustand des Reaktorinhalts werden im Sinne der Erfindung durch mindestens eine Regelungseinheit ausgeführt oder implementiert. Insofern sind Regelungseinheiten alle Vorrichtungen, welche Permittivitätssignale oder aus ihnen abgeleitete Werte sowie Daten zu Form, Verteilung oder Bewegungszustand des Reaktorinhalts mit der Planung, Durchführung, Verarbeitung, Auswertung oder Visualisierung der abzustimmenden optischen Messung in Zusammenhang bringen. Je nach Abstimmungsverfahren sind Regelungseinheiten im Sinne der Erfindung insbesondere aber nicht ausschließlich Operationsverstärker, Komparatoren, PID-Regler, Schmitt-Trigger und Rechner. Als Rechner zählt jede elektronische Vorrichtung, die Daten (insbesondere arithmetische und logische) speichern und auf der Grundlage programmierbarer Vorschriften verarbeiten kann. Als Rechner im Sinne der Erfindung gelten insbesondere aber nicht ausschließlich Mikrocontroller, Mikroprozessoren, System-on-a-Chip Rechnern (SoC), PCs und Server.

Erfindungsgemäß ist mindestens eine optische Messung auf die Form, Verteilung oder den Bewegungszustand des Reaktorinhalts abzustimmen. Die mindestens eine optische Messung erfolgt dabei unter Verwendung mindestens einer optischen Messanordnung, zumeist, aber nicht notwendigerweise, umfassend, insbesondere bestehend aus, mindestens eine Lichtquelle, welche Licht in den Reaktorinhalt einstrahlt und mindestens einem Lichtsensor, welcher Licht aus dem Reaktorinhalt erfasst.

Erfindungsgemäß umfasst der Reaktorinhalt mindestens zwei, nicht vollständig mischbaren Phasen, wobei sich mindestens zwei Phasen in ihrer phasenspezifischen Permittivität unterscheiden. Der Reaktorinhalt kann insbesondere aus diesen beiden Phasen bestehen,

Typische mehrphasige Systeme mit lokal unterschiedlichen Permittivitäten entstehen insbesondere, aber nicht ausschließlich, wenn der Reaktorinhalt Phasen verschiedener Aggregatszustände enthält, beispielsweise Luft und wässrige Lösungen oder Suspensionen, oder wässrige Lösungen und bewegte Festkörper (z.B. Rührer). Ebenfalls werden mehrphasige Systeme gebildet durch nicht vollständig mischbare Phasen gleichen Aggregatszustandes (z.B. Emulsionen).

Die Durchmischung des Reaktorinhalts und die damit verbundene Bewegung, Form-, oder Verteilungsänderung des Reaktorinhalts relativ zum Reaktor führt insbesondere zu einer damit einhergehenden Änderung der lokalen Permittivität an mindestens einem Ort innerhalb des Reaktors. Insofern ist diese lokale Permittivität, ihre Änderung sowie weitere von ihr abgeleitete Werte ein Maß für die Form, die Verteilung oder den Bewegungszustand des Reaktorinhalts oder mindestens einer seiner Phasen zum Zeitpunkt ihrer Erfassung.

Die zur Abstimmung optischer Messungen an kontinuierlich durchmischten Reaktoren notwendige Erfassung der Form, der Verteilung oder des Bewegungszustandes des Reaktorinhalts in Bezug auf die abzustimmende optische Messanordnung wird erfindungsgemäß erreicht durch die ortsaufgelöste Erfassung lokaler Permittivitäten des Reaktorinhalts. Dadurch erfolgt in Abgrenzung zum Stand der Technik eine direkte Erfassung der Form, der Verteilung oder des Bewegungszustandes des Reaktorinhalts, welche den aus dem Stand der Technik bekannten Verfahren der indirekten Erfassung der Form, der Verteilung oder des Bewegungszustandes des Reaktorinhalts über Beschleunigungsmessungen des Reaktors oder Positionsmessungen des Reaktors überlegen ist. Im Gegensatz zu aus dem Stand der Technik bekannten Verfahren zur optischen Abschätzung der Form, der Verteilung oder des Bewegungszustandes des Reaktorinhalts ist das erfindungsgemäße Verfahren besser geeignet zur Abstimmung optischer Messungen, da es insbesondere nicht selbst den Fehleranfälligkeiten sämtlicher optischer Messverfahren an kontinuierlich durchmischten Reaktoren unterliegt.

Die erfindungsgemäße Erfassung mindestens einer lokalen Permittivität des Reaktorinhalts erfolgt in einem bekannten Abstand zu mindestens einer optischen Messanordnung.

Das erfindungsgemäß durch mindestens einen Permittivitätssensor erfasste Permittivitätssignal mindestens eines Ortes mit bekanntem Abstand zur mindestens einen abzustimmenden optischen Messanordnung wird von einer Regelungseinheit genutzt, um mindestens eine optische Messung auf die Form, die Verteilung oder den Bewegungszustand des Reaktorinhalts oder mindestens einer seiner Phasen abzustimmen, wobei sich das erfindungsgemäße Verfahren die direkte Korrelation von lokaler Permittivität an einem bestimmten Ort oder Raum innerhalb des Reaktors mit der Form, Verteilung oder dem Bewegungszustand des Reaktorinhalts oder mindestens einer seiner Phasen zunutze macht.

Die Erfassung mindestens einer lokalen Permittivität erfolgt kontinuierlich oder zu diskreten Zeitpunkten, in vorteilhafter Ausgestaltung der Erfindung mit einer Frequenz, die höher ist als die Durchmischungsfrequenz des Reaktors oder mit einer Frequenz, sodass die Durchmischungsfrequenz des Reaktors kein ganzzahliges Vielfaches der Erfassungsfrequenz ist.

In vorteilhafter Ausgestaltung der Erfindung wird mindestens eine lokale Permittivität oder ihre Änderung durch einen Permittivitätssensor erfasst, welcher sich in einem elektrischen Feld befindet, dessen Stärke, Form und Richtung sich in Abhängigkeit der lokalen Permittivität und damit in Abhängigkeit der Form, der Verteilung und des Bewegungszustandes des Reaktorinhalts ändert. Vorteilhaft einsetzbare erfindungsgemäße Permittivitätssensoren sind daher alle Arten kapazitiver Sensoren. Erfindungsgemäß anwendbare Messverfahren umfassen dabei insbesondere, aber nicht ausschließlich absolute und vergleichende Charge-Transfer-Methoden, amplitudenmodulierte Methoden zur Erfassung des Blindstroms über den kapazitiven Sensor sowie frequenzmodulierte Methoden, welche Verschiebungen in der Resonanzfrequenz eines mit dem Sensor gekoppelten Schwingkreises oder sonstigen Oszillators erfassen.

Erfindungsgemäß anwendbare Permittivitätssensoren umfassen in einigen Ausführungsformen eine, zwei, oder mehrere interagierenden oder auf sich selbst bezogenen Elektroden zur Ein- und Auskopplung elektrischer Felder zum Zweck der Permittivitätserfassung.

In vorteilhafter Ausgestaltung der Erfindung erfolgt zwischen Reaktor und mindestens einem Permittivitätssensor keine Relativbewegung.

In vorteilhafter Ausgestaltung der Erfindung erfolgt zwischen Reaktor und mindestens einer optischen Messanordnung keine Relativbewegung.

In vorteilhafter Ausgestaltung der Erfindung erfolgt zwischen mindestens einem Permittivitätssensor und mindestens einer optischen Messanordnung keine Relativbewegung.

In vorteilhafter Ausgestaltung der Erfindung befindet sich mindestens ein Permittivitätssensor außerhalb des Reaktors und ermöglicht insbesondere somit die nichtinvasive Erfassung der lokalen Permittivität und damit der Form, Verteilung oder des Bewegungszustandes des Reaktorinhalts.

In vorteilhafter Ausgestaltung der Erfindung ist mindestens ein Permittivitätssensor derart elektrisch geschirmt, dass er nur Permittivitätsänderungen des Reaktorinhalts erfasst. Insbesondere ist eine derartige Schirmung vorteilhaft zur Reduktion oder Eliminierung störender externer elektrischer Felder, beispielsweise vom elektrischen Antrieb eines Schüttlers oder von durch funkende Geräte ausgesendeten elektrischen Feldern.

In vorteilhafter Ausgestaltung der Erfindung ist das sensorische elektrische Feld durch geeignete Vorrichtungen formbar, um die Ortsauflösung oder Sensitivität des Permittivitätssensors anzupassen und zu regeln.

Bei Anwendung modulierter Verfahren zur Erfassung lokaler Permittivitäten ist in vorteilhafter Ausgestaltung der Erfindung mindestens einer der modulierten Parameter im Laufe der Zeit änderbar, um insbesondere Änderungen der lokalen Permittivität, welche nicht durch die Durchmischung des Reaktorinhalts verursacht wurden, auszugleichen oder ihnen anderweitig Rechnung zu tragen.

Bei Anwendung von Verfahren zur Erfassung lokaler Permittivitäten, die sich gepulster Spannungen oder Ströme oder anderweitiger Wechselspannungen oder Wechselströme bedienen, ist in vorteilhafter Ausgestaltung der Erfindung der Frequenzbereich der Puls- oder Wechselfrequenz derart regelbar oder wählbar, insbesondere dass solche chemischen Substanzen, insbesondere Ionen und polare Moleküle, die nicht eine Hauptkomponente oder ein Hauptmedium des Reaktorinhalts sind, nicht die Erfassung lokaler Permittivitäten beeinflussen. Vorteilhaft einsetzbar ist hier die Frequenzabhängigkeit der Permittivität.

In vorteilhafter Ausgestaltung der Erfindung setzt sich der Reaktorinhalt zu mindestens zwei Dritteln aus maximal zwei Hauptkomponenten zusammen, deren komponentenbezogene Permittivität über die gesamte Zeit des im Reaktor ablaufenden Prozesses nahezu oder vollständig konstant bleibt. Ein typisches Beispiel dafür ist die Kultivierung von Zellen, bei der der Reaktorinhalt zu mehr als zwei Dritteln aus flüssigem Wasser und Luft oder einer sonstigen Gasphase besteht.

In einer Ausgestaltung der Erfindung ist die im Laufe eines im Reaktor ablaufenden Prozesses sich ändernde Stoffzusammensetzung einer jeden Phase und die damit verbundene Änderung der phasenspezifischen Permittivität durch Kalibrations- oder Driftmessungen erfassbar und die Erfassung der Form, der Verteilung oder des Bewegungszustandes des Reaktorinhalts durch diese Werte anpass- oder korrigierbar.

In vorteilhafter Ausgestaltung der Erfindung können zusätzliche lokale Messdaten anderer Sensoren in die Erfassung der Form, der Verteilung oder des Bewegungszustandes des Reaktorinhalts einbezogen werden, insbesondere aber nicht ausschließlich Daten von Beschleunigungssensoren, Positionssensoren und optischen Sensoren.

In vorteilhafter Ausgestaltung der Erfindung umfasst das beanspruchte Verfahren Methoden zur Vorhersage der Form, der Verteilung oder des Bewegungszustandes des Reaktorinhalts, insbesondere aber nicht ausschließlich Kalman-Filter und vergleichbare bayessche Schätzverfahren, sowie Regressions- und Extrapolationsmethoden.

In vorteilhafter Ausgestaltung der Erfindung wird die jeweils lokale Permittivität an zwei oder mehr Positionen mit bekanntem Abstand zu mindestens einer abzustimmenden optischen Messanordnung erfasst, sodass sich insbesondere ein detailreicheres Bild der Form, der Verteilung oder des Bewegungszustandes des Reaktorinhalts ergibt und die Abstimmung mindestens einer optischen Messung genauer erfolgen kann.

In vorteilhafter Ausgestaltung der Erfindung kann die Form, die Verteilung oder der Bewegungszustand mindestens einer Phase des Reaktorinhalts über mindestens einer optischen Messanordnung in die Auswertungsrechnungen und Analyse der Messdaten der jeweiligen optischen Messanordnung einbezogen werden. Dies ermöglicht insbesondere die Korrektur der optischen Messdaten bezüglich kleiner Positionierungsunterschiede von Reaktor und der jeweiligen optischen Messanordnung zwischen zwei oder mehr ansonsten gleich aufgebauten erfindungsgemäßen Messanordnungen.

In vorteilhafter Ausgestaltung der Erfindung kann über die Erfassung der zeitabhängigen Änderung mindestens einer lokalen Permittivität die Bildung oder das Verschwinden neuer Phasen oder Phasensysteme erfasst werden, beispielsweise die Bildung von Schäumen oder wässrig-öligen Emulsionen.

In vorteilhafter Ausgestaltung der Erfindung kann über die Erfassung der zeitabhängigen Änderung mindestens einer lokalen Permittivität die Zunahme oder Abnahme des Volumenanteils mindestens einer Phase des Reaktorinhalts erfasst werden, welche insbesondere als Folge der Zugabe von Substanzen (Feeding, pH-Regelung, Induktionsmittel, Antischaummittel) oder als Folge der Entnahme von Reaktorinhaltsbestandteilen (Sampling, Teilernte) sowie als Folge von Aggregatszustandsänderungen (z.B. Evaporation, Auflösung, Ausfällung, Kristallisation, Schmelzen, Kondensation, etc.) und gegebenenfalls damit verbundenem Entweichen aus dem Reaktor zu beobachten sind.

In vorteilhafter Ausgestaltung der Erfindung ermöglicht die Erfassung der zeitabhängigen Änderung mindestens einer lokalen Permittivität die Beurteilung oder Ableitung weiterer Parameter des Reaktorinhalts, insbesondere, aber nicht ausschließlich der Viskosität und anderer fluidmechanischer Parameter.

Die vorliegende Erfindung wird anhand der Figuren und Ausführungsbeispiele näher erläutert.

Ausführungsbeispiele und Figuren
- Figur 1,: eine schematische Blockdarstellung des erfindungsgemäßen Verfahrens unter Verwendung einer erfindungsgemäßen Vorrichtung.
- Figur 3,: eine schematische Darstellung einer erfindungsgemäßen Vorrichtung zur Durchführung des erfindungsgemäßen Verfahrens an einem Schüttelkolben als Reaktor zur Kultivierung von Zellen.

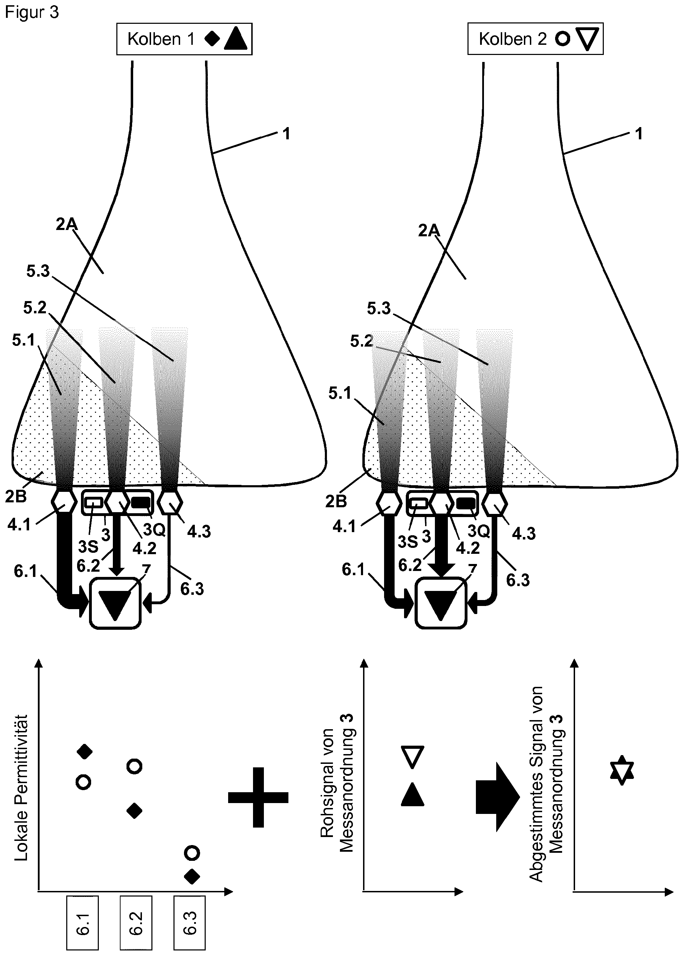

- Figur 3,: eine schematische Darstellung einer erfindungsgemäßen Vorrichtung zur Durchführung des erfindungsgemäßen Verfahrens mit mehreren Permittivitätssensoren.

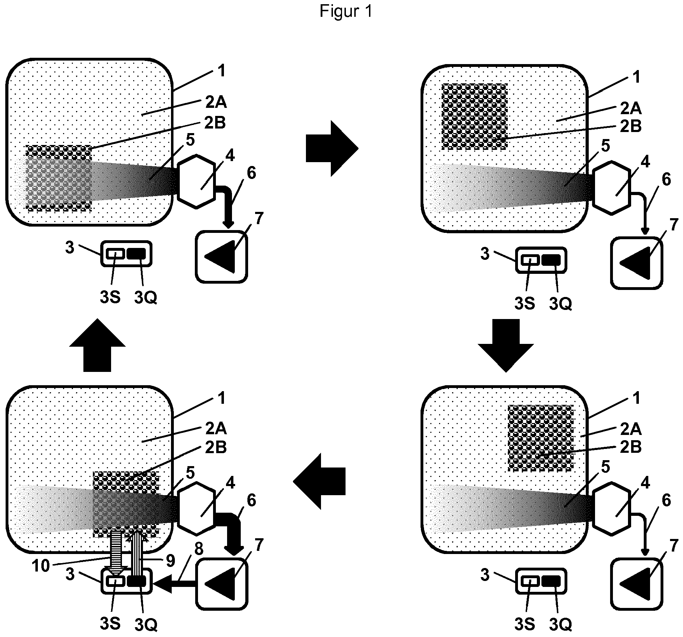

Figur 1 zeigt eine schematische Blockdarstellung des erfindungsgemäßen Verfahrens unter Verwendung einer erfindungsgemäßen Vorrichtung. Die Vorrichtung umfasst einen Reaktor **1** mit einem Reaktorinhalt **2.** In der schematischen Darstellung der Figur 1 umfasst der Reaktorinhalt **2** zwei nicht vollständig mischbare Phasen **2A** und **2B** unterschiedlicher phasenspezifischer Permittivität, insbesondere setzt sich der Reaktoinhalt aus den beiden Phasen zusammen. Figur 1 zeigt vier Momentaufnahmen aus einer kontinuierlichen Durchmischungsbewegung des Reaktors **1,** welche in ihrem Verlauf eine Veränderung der Form, Verteilung oder des Bewegungszustandes des Reaktorinhalts **2** bedingt. Außerhalb des Reaktor **1** befindet sich mindestens eine optische Messanordnung **3,** deren Messungen auf Form, Verteilung oder Bewegungszustand des Reaktorinhalts **2** abzustimmen sind. In vorteilhafter Ausgestaltung der Erfindung umfasst mindestens eine optische Messanordnung **3** mindestens eine Lichtquelle **3Q** zur Einstrahlung von Licht in den Reaktorinhalt **2** und/oder mindestens einen Lichtsensor **3S** zur Erfassung von aus dem Reaktorinhalt **2** ausgestrahltem Licht. Über mindestens einen Permittivitätssensor **4,** welcher mit mindestens einem elektrischen Feld **5** interagiert, erfolgt die Erfassung der lokalen Permittivität in einem bestimmten Bereich des Reaktorinhalts **2,** resultierend in mindestens einem Permittivitätssignal **6,** welches an eine Regelungseinheit **7** weitergeleitet wird. Diese wiederum übernimmt die Abstimmung **8** mindestens einer optischen Messanordnung **3.** In einigen Ausführungsformen der Erfindung kann die Abstimmung **8** ein Aktivierungssignal für mindestens eine optische Messanordnung **3** sein, so auch in Figur 1.

Das erfindungsgemäße Verfahren wird aus Figur 1 ersichtlich. Durchmischungsbedingt ändert sich die lokale Permittivität im Reaktor **1,** wodurch vermittels des mit Reaktorinhalt **2** und Permittivitätssensor **4** interagierenden elektrischen Feldes **5** und der damit einhergehenden Erfassung der lokalen Permittivität im Interaktionsbereich des Permittivitätssensors **4** Informationen über die Form, die Verteilung oder den Bewegungszustand des Reaktorinhalts **2** in Form eines Permittivitätssignals **6** an die Regelungseinheit **7** übertragen werden und durch diese eine Abstimmung der optischen Messungen mindestens einer optischen Messanordnung **3** erfolgt.

Für die in Figur 1 dargestellte Ausführungsform der Erfindung erfolgt die Abstimmung **8** insbesondere als Aktivierung der optischen Messanordnung **3,** wenn sich Reaktorinhalt **2** mit dominanter Phase **2B** vor der optischen Messanordnung **3** befindet. Die mit diesem Bewegungs- und Verteilungszustand einhergehende Änderung der lokalen Permittivität resultiert in einem starken Permittivitätssignal **6,** infolgedessen die Regelungseinheit **7** die optische Messanordnung **3** aktiviert (Abstimmung **8,** Teildarstellung unten links). In der dargestellten Ausführungsform erfolgt die Abstimmung **8** durch Aktivierung mindestens einer Lichtquelle **3Q** der abzustimmenden optischen Messanordnung **3,** sodass nun Licht **9** von der Lichtquelle **3Q** in den Reaktorinhalt **2,** insbesondere aber in die Phase **2B** eingestrahlt wird, wo es mit Komponenten des Reaktorinhalts **2** interagiert und nachfolgend als auf den Lichtsensor **3S** eingehendes Licht **10** detektiert wird. Damit ist die optische Messung der optischen Messanordnung **3** auf die Form, Verteilung oder den Bewegungszustand des Reaktorinhalts **2** abgestimmt, in der dargestellten Ausführungsform insbesondere durch Synchronisation der optischen Messung auf die Anwesenheit der Phase **2B** vor der optischen Messanordnung **3.**

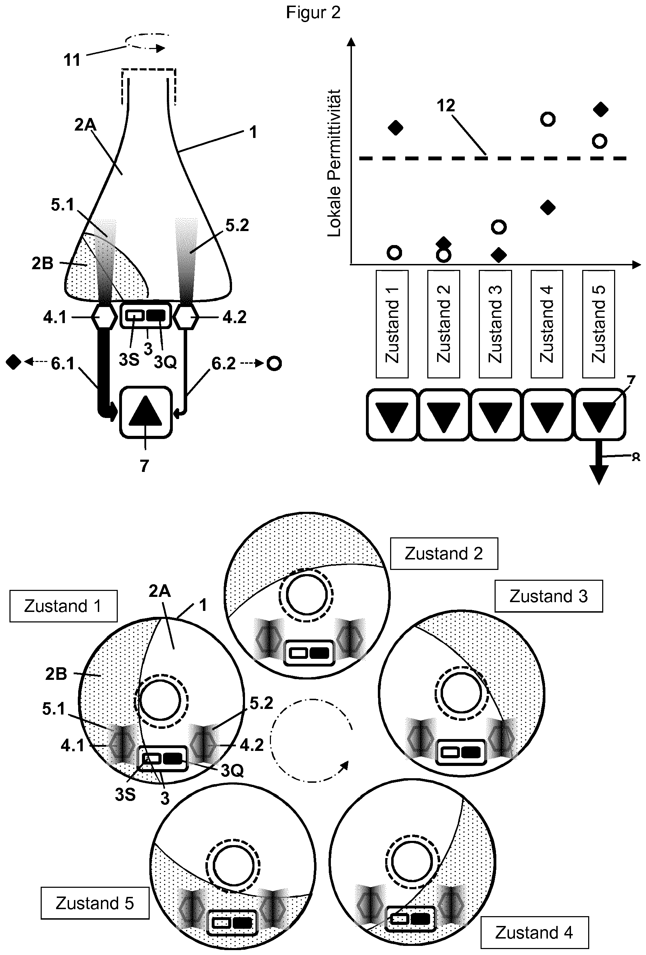

Figur 2 zeigt eine schematische Darstellung einer erfindungsgemäßen Vorrichtung zur Durchführung des erfindungsgemäßen Verfahrens an einem Schüttelkolben als Reaktor **1** zur Kultivierung von Zellen. Der obere linke Teil der Figur 2 zeigt eine Seitenansicht der erfindungsgemäßen Messanordnung. Im unteren Teil der Figur 2 sind fünf exemplarische Zustände der erfindungsgemäßen Ausführungsform im Verlauf der Schüttelbewegung **11** als Orbitalschüttelbewegung dargestellt. Gezeigt ist dabei eine schematische Ansicht der Ausführungsform von oben. Im oberen rechten Teil der Figur 2 ist ein schematisches Diagramm gezeigt, welches für jeden der unten aufgeführten fünf Bewegungszustände das jeweils zugehörige Permittivitätssignal **6.1/6.2** darstellt.

Als Reaktor **1** kommt ein Schüttelkolben zum Einsatz. Der Reaktorinhalt **2** umfasst zwei Phasen **2A** und **2B,** wobei **2A** als gasförmige Phase durch Luft gebildet wird und sich **2B** als insgesamt flüssige Phase überwiegend aus Wasser, sowie aus verschiedenen Kulturmedienbestandteilen und Zellen zusammensetzt (Kulturbrühe als Suspension aus Zellen in Kulturmedium). Zur Durchmischung des Reaktorinhalts **2** führt der Kolben als Reaktor **1** eine Schüttelbewegung **11** durch, welche für eine fortlaufende Veränderung von Form, Verteilung und Bewegungszustand des Reaktorinhalts **2** verantwortlich ist.

Unter dem Reaktor **1** befindet sich eine optische Messanordnung **3,** deren Messungen auf die Form, Verteilung oder den Bewegungszustand des Reaktorinhalts **2** abzustimmen sind. Optische Messanordnungen im Sinne der Erfindung und im Anwendungsbereich der in Figur 2 dargestellten Ausgestaltung der Erfindung sind insbesondere aber nicht ausschließlich Messanordnungen zur Erfassung der Trübung, Zelldichte oder Biomassekonzentration der Phase **2B** über Streulicht- oder Absorptionsmessungen, sowie Messanordnungen zur Anregung und/oder Erfassung von Fluoreszenz oder Biolumineszenz bestimmter Bestandteile von Phase **2B** oder bestimmter Fluoreszenz-/Lumineszenz-Sensoren, welche mit Phase **2B** zumindest zeitweilig in Kontakt stehen (z.B. GFP, NADH, Proteine, Luciferin, fluoreszierende pH-, Sauerstoff, CO₂-, Glucose-, Lactat- oder Metallionen-Sensoren).

Die optische Messanordnung **3** ist tangential zur Schüttelbewegung flankiert durch zwei Permittivitätssensoren **4.1/4.2,** die lokal über ein jeweils zugehöriges elektrisches Feld **5.1/5.2** mit dem Reaktorinhalt **2** und seinen Bestandteilen interagieren. In vorteilhafter Ausgestaltung der Erfindung sind, wie in Figur 2 dargestellt, mindestens zwei Permittivitätssensoren **4.1/4.2** entlang des Bewegungspfades des durchmischten Reaktorinhalts **2** angeordnet. Die Permittivitätssensoren **4.1/4.2** erfassen lokale Permittivitätsänderungen in ihrer Nähe, welche durch die Bewegung des Reaktorinhalts **2** und insbesondere seiner Phasen **2A** und **2B** verursacht werden und erlauben somit eine Beurteilung der lokalen Form, Verteilung oder des Bewegungszustandes des Reaktorinhalts **2.** Die resultierenden Permittivitätssignale **6.1** (gefüllte Raute) und **6.2** (Kreisring) werden an eine Regelungseinheit **7** weitergegeben, welche die Abstimmung **8** der Messungen der optischen Messanordnung **3** vornimmt.

Die Umsetzung des erfindungsgemäßen Verfahrens erfolgt in der Ausführungsform der Figur 2 folgendermaßen. Innerhalb einer Schüttelperiode erfassen die Permittivitätssensoren **4.1/4.2** mehrmals (hier exemplarisch fünfmal) die lokale Permittivität des Reaktorinhalts **2.** Da die wässrige Phase **2B** eine deutlich höhere relative Permittivität aufweist als die Luft der gasförmigen Phase **2A,** ergibt sich infolge der Bewegung der Flüssigkeit für jeden Permittivitätssensor **4.1/4.2** ein charakteristisches Permittivitätssignal **6.1/6.2** in Abhängigkeit des jeweiligen Bewegungszustandes des Reaktorinhalts **2.** Je größer der lokale Anteil an Phase **2B** im Interaktionsbereich des jeweiligen elektrischen Feldes **5.1/5.2** ist, umso höher ist das Permittivitätssignal **6.1/6.2** des jeweiligen Permittivitätssensors **4.1/4.2.** Aufgrund der bekannten Abstände zwischen optischer Messanordnung **3** und den Permittivitätssensoren **4.1/4.2** kann die Regelungseinheit **7** die Form, Verteilung oder den Bewegungszustand des Reaktorinhalts **2** in räumlichem Bezug auf die optische Messanordnung **3** erfassen, insbesondere, wann sich wieviel wässrige Phase **2B** über der optischen Messanordnung **3** befindet.

Die resultierenden bewegungsabhängigen lokalen Permittivitätssignale **6.1/6.2** sind im Diagramm in Figur 2 oben rechts schematisch dargestellt. Erfolgt die Abstimmung **8** der optischen Messanordnung **3** als Aktivierung oder Deaktivierung, so kann in vorteilhafter Ausgestaltung der Erfindung die Einführung mindestens einer Grenzbedingung **12** nutzbringend eingesetzt werden, wobei das Aktivierungssignal als Form der Abstimmung **8** nur dann durch die Regelungseinheit **7** ausgegeben wird, wenn die lokalen Permittivitätssignale **6.1/6.2** die Grenzbedingung **12** erfüllen. Die in Figur 2 dargestellte Grenzbedingung **12** wird durch eine Linie gebildet, über der die lokalen Permittivitätssignale **6.1/6.2** liegen müssen, damit eine Aktivierung **8** der optischen Messanordnung **3** durch die Regelungseinheit **7** erfolgt. Diese Grenzbedingung ist in der abgebildeten Ausführungsform der Erfindung nur durch Zustand 5 erfüllt, sodass eine Aktivierung **8** der optischen Messanordnung **3** nur dann erfolgt, wenn sie vollständig mit Phase **2B** (Kulturmedium mit Zellen) mit einer bestimmten Mindesthöhe bedeckt ist. Somit ist eine Abstimmung **8** der Messungen der optischen Messanordnung **3** auf die Form, Verteilung und den Bewegungszustand des Reaktorinhalts **2** und insbesondere der wässrigen Phase **2B** durch Erfassung zweier lokaler Permittivitäten in einem bekannten Abstand zur optischen Messanordnung **3** erreicht.

Eine derartige Abstimmung optischer Messungen kann in kontinuierlich geschüttelten Reaktorsystemen (Schüttelkolben, aber auch Mikrotiterplatten, Schüttelfässer, T-Flasks und Schüttelsäcke) insbesondere aber nicht ausschließlich nutzbringend angewendet werden, um optische Messungen direkt auf den maximalen Flüssigkeitsstand über mindestens einer optischen Messanordnung abzustimmen und somit externe Störfaktoren wie Schaum, inhomogene Phasenverteilungen oder unerwünschte Reflektionen und Streuungen an der Reaktorwand zu eliminieren oder zu reduzieren. Die direkte Erfassung der Form, Verteilung oder des Bewegungszustandes des Reaktorinhalts über die Erfassung lokaler Permittivitäten oder ihrer Änderung ist dabei den aus dem Stand der Technik bekannten Methoden hinsichtlich Genauigkeit, zeitlicher und räumlicher Auflösung, Berechnungsaufwand und Anfälligkeit für prozessbegleitende Änderungen der Fluidcharakteristik des Reaktorinhalts 2 überlegen.

Figur 3 zeigt eine schematische Darstellung einer erfindungsgemäßen Vorrichtung zur Durchführung des erfindungsgemäßen Verfahrens mit mehreren Permittivitätssensoren. Dargestellt sind zwei identische erfindungsgemäße Vorrichtungen mit zwei Schüttelkolben als Reaktor **1,** wobei deren Positionierung über der optischen Messanordnung **3** und den Permittivitätssensoren **4.1/4.2/4.3** unterschiedlich ist.

Bezüglich der Zusammensetzung des Reaktorinhalts **2,** der Durchmischungsbewegung, der grundsätzlichen Interaktion der Permittivitätssensoren **4.1/4.2/4.3** mit dem Reaktorinhalt **2** und der Charakteristik der optischen Messanordnung **3** entspricht die in Figur 3 dargestellte Ausführungsform der Erfindung den Beschreibungen aus Figur 2. Abweichend davon befinden sich jedoch drei Permittivitätssensoren **4.1/4.2/4.3** in bekannten Abständen zur optischen Messanordnung **3,** wobei die Permittivitätssensoren **4.1/4.3** diese radial zur Schüttelbewegung flankieren und Permittivitätssensor **4.2** zwischen Lichtquelle **3Q** und Lichtsensor **3S** angeordnet ist. Infolge der radialen Anordnung erfassen die Permittivitätssensoren **4.1/4.2/4.3** einen radialen Volumenbereich des Reaktorinhalts **2,** sodass die jeweiligen Permittivitätssignale **6.1/6.2/6.3** ein Höhenprofil oder Volumenprofil eines radialen Schnittes durch den Reaktorinhalt **2** wiedergeben. Dies ist in Figur 3 im linken unteren Diagramm dargestellt für den in Figur 3 oben gezeigten Bewegungs- und Verteilungszustand des Reaktorinhalts **2,** wobei die Permittivitätssignale **6.1/6.2/6.3** für Kolben 1 als gefüllte Raute und für Kolben 2 als Kreisring dargestellt sind.

Der in Figur 3 dargestellte Bewegungszustand entspricht dem Zustand 5 aus Figur 2, bei dem eine Aktivierung **8** der optischen Messanordnung **3** erfolgt. Wie aus Figur 3 hervorgeht, verändert sich infolge der relativen Positionierungsunterschiede zwischen optischer Messanordnung **3** und Reaktor **1** sowie Reaktorinhalt **2** das Volumen der für die optische Messung relevanten Phase **2B** im Sichtfeld der optischen Messanordnung, was wiederum, trotz gleicher Bedingungen in Phase **2** (z.B. gleiche Zelldichte), zu Unterschieden im Signal der optischen Messanordnung **3** führt (siehe Diagramm unten mittig in Figur 3, ausgefülltes Dreieck für Kolben 1, leeres Dreieck für Kolben 2).

Die erfindungsgemäße Abstimmung **8** optischer Messungen an kontinuierlich durchmischten Reaktoren **1** umfasst daher auch die Korrektur optischer Signale mittels der durch die lokalen Permittivitätsmessungen erhaltenen Permittivitätssignale **6.1/6.2/6.3** und die aus ihnen abgeleiteten Informationen über die Form, Verteilung oder den Bewegungszustand des Reaktorinhalts **2.** Wie in Figur 3, Diagramm unten links, dargestellt, ergeben sich je nach Positionierung oder Füllvolumen des Reaktor **1** unterschiedliche lokale Permittivitätsprofile. Diese können erfindungsgemäß insbesondere aber nicht ausschließlich dazu genutzt werden, Volumenprofile abzuleiten und daraus das sich im Sichtfeld der optischen Messanordnung befindliche Volumen der für die optische Messung relevanten Bestandteile des Reaktorinhalts **2** (in Figur 3 als Phase **2B**) zu berechnen. Auf der Grundlage derartiger Berechnungen erfolgt dann eine korrigierende Abstimmung und insbesondere Normierung der optischen Messung auf das messungsrelevante Volumen im Sichtfeld der optischen Messanordnung **3** durch die Regelungseinheit **7.** Das Ergebnis ist ein von Form, Verteilung oder Bewegungszustand unabhängiges, abgestimmtes Messsignal der jeweiligen optischen Messanordnung 3, wie in Figur 3, unten rechts dargestellt (ausgefülltes Dreieck für Kolben 1, leeres Dreieck für Kolben 2, beide überlappend, da abgestimmt bei gleichem Y-Achsen-Wert).

Derartige normierende Abstimmungen optischer Messungen an kontinuierlich durchmischten Reaktoren **1** finden erfindungsgemäß insbesondere Anwendung zur Abstimmung auf Positionierungsunterschiede zwischen mindestens zwei zu vergleichenden Reaktoren **1,** zur Abstimmung auf unterschiedliche Füllvolumina oder Phasenanteile der Reaktorinhalte **2** mindestens zweier zu vergleichender Reaktoren **1,** sowie zur Abstimmung auf unterschiedliche Durchmischungsbedingungen und damit Unterschiede in Form, Verteilung oder Bewegungszustand der Reaktorinhalte **2** mindestens zweier zu vergleichender Reaktoren **1.**

**Bezugszeichenliste**

| Zur jeweiligen Interpretation der Bezugszeichen sind Beschreibung und Ansprüche zu beachten. | |
|---|---|
| **1** | Reaktor |
| **2, 2A, 2B** | Reaktorinhalt und Phasen 2A, 2B mit unterschiedlichen phasenspezifischen Permittivitäten |
| **3, 3Q, 3S** | Optische Messanordnung mit Lichtquelle 3Q und Lichtsensor 3S |
| **4, 4.1, 4.2, 4.3** | Permittivitätssensor, mehrere Permittivitätssensoren 4.1, 4.2, 4.3 |
| **5, 5.1, 5.2, 5.3** | Mit Permittivitätssensor 4 und Reaktorinhalt 2 interagierendes elektrisches Feld, mehrere elektrische Felder 5.1, 5.2, 5.3 interagierend jeweils Reaktorinhalt 2 und mit Permittivitätssensor 4.1, 4.2, 4.3 |
| **6, 6.1, 6.2, 6.3** | Permittivitätssignal, Signale 6.1, 6.2, 6.3 der jeweiligen Permittivitätssensoren 4.1, 4.2, 4.3 |
| **7** | Regelungseinheit |
| **8** | Abstimmung mindestens einer optischen Messanordnung 3 |
| **9** | Ausgehendes Licht aus Lichtquelle 3Q |
| **10** | Eingehendes Licht auf Lichtsensor 3S |
| **11** | Bewegung zur Durchmischung des Reaktorinhalts 2 |
| **12** | Grenzbedingung zur Aktivierung (als Abstimmung 8) der optischen Messanordnung 3 |

## Patentansprüche

1. Verfahren zur Abstimmung (8) optischer Messungen an kontinuierlich durchmischten Reaktoren (1),
wobei ein Reaktorinhalt (2) des Reaktors (1) mindestens eine optische erfassbare Messgröße aufweist,
wobei mindestens eine optische Messanordnung (3) optische Messungen mindestens einer der optisch erfassbaren Messgrößen durchführen kann, insbesondere durchführt,
wobei mindestens eine optische Messung auf die Form, die Verteilung oder den Bewegungszustand mindestens einer Phase (2A, 2B) des Reaktorinhalts (2) abzustimmen ist, insbesondere abgestimmt wird,
wobei die Durchmischung des Reaktorinhalts (2) lokale Änderungen der Permittivität innerhalb des Reaktors (1) hervorruft,
**dadurch gekennzeichnet,**
**dass** an mindestens einem Ort mit bekanntem Abstand zur mindestens einen abzustimmenden optischen Messanordnung (3) die lokale Permittivität, insbesondere anhand eines Permittivitätssignals (6), erfasst wird, und
**dass** das erfasste Permittivitätssignal (6) mindestens eines Ortes mit bekanntem Abstand zur mindestens einen abzustimmenden optischen Messanordnung (3) genutzt wird, um mindestens eine optische Messung auf die Form, die Verteilung oder den Bewegungszustand des Reaktorinhalts (2) abzustimmen.

2. Verfahren nach dem vorherigen Anspruch,
**dadurch gekennzeichnet,**
**dass** die Änderung mindestens einer lokalen Permittivität oder sonstige aus ihr abgeleiteten Werte genutzt werden, um mindestens eine optische Messung auf die Form, die Verteilung oder den Bewegungszustand mindestens einer Phase (2A, 2B) des Reaktorinhalts (2) abzustimmen.

3. Verfahren nach einem der vorherigen Ansprüche,
**dadurch gekennzeichnet,**
**dass** die Abstimmung (8) mindestens einer optischen Messanordnung (3) als Aktivierung und Deaktivierung der optischen Messanordnung (3) in Abhängigkeit der Form, Verteilung oder des Bewegungszustands mindestens einer Phase (2A, 2B) des Reaktorinhalts (2) in einem Sichtfeld der optischen Messanordnung (3) erfolgt.

4. Verfahren nach einem der vorherigen Ansprüche,
**dadurch gekennzeichnet,**
**dass** die Abstimmung (8) mindestens einer optischen Messanordnung (3) als Korrektur oder Normierung des optischen Messsignals der optischen Messanordnung (3) in Abhängigkeit der Form, Verteilung oder des Bewegungszustands mindestens einer Phase des Reaktorinhalts (2) in einem Sichtfeld der optischen Messanordnung (3) erfolgt.

5. Verfahren nach einem der vorherigen Ansprüche,
**dadurch gekennzeichnet,**
**dass** die Erfassung der Form, Verteilung oder des Bewegungszustands des Reaktorinhalts (2) durch mehrere Permittivitätssensoren (4.1, 4.2, 4.3) erfolgt.

6. Verfahren nach einem der vorherigen Ansprüche,
**dadurch gekennzeichnet,**
**dass** die Erfassung mindestens einer lokalen Permittivität über mindestens ein elektrisches Feld (5) erfolgt, welches lokal mit dem Reaktorinhalt (2) und mindestens einem Permittivitätssensor (4) interagiert.

7. Verfahren nach einem der vorherigen Ansprüche,
**dadurch gekennzeichnet,**
**dass** die Erfassung mindestens einer lokalen Permittivität über ein kapazitives Messverfahren, insbesondere anhand eines kapazitiven Permittivitätssensors (4), erfolgt.

8. Vorrichtung zur Durchführung des Verfahrens nach einem der vorherigen Ansprüche,
**dadurch gekennzeichnet,**
**dass** die Vorrichtung an mindestens einem Ort mit bekanntem Abstand zur mindestens einen abzustimmenden optischen Messanordnung (3) mindestens einen Permittivitätssensor (4) umfasst, welcher eingerichtet ist, Änderungen der lokalen Permittivität zu erfassen, und
**dass** die Vorrichtung mindestens eine Regelungseinheit (7) umfasst, welche eingerichtet ist, auf Basis mindestens eines Permittivitätssignals (6) mindestens eines Permittivitätssensors (4) die Abstimmung der optischen Messung auf die Form, die Verteilung oder den Bewegungszustand des Reaktorinhalts oder mindestens einer seiner Phasen vorzunehmen.

9. Vorrichtung nach dem vorherigen Anspruch,
**dadurch gekennzeichnet,**
**dass** die Regelungseinheit (7) eingerichtet ist, ein Verfahren nach einem der Ansprüche 1 bis 7 zu steuern.

10. Vorrichtung nach einem der Ansprüche 8 bis 9,
**dadurch gekennzeichnet,**
**dass** die Vorrichtung mindestens einen kapazitiven Permittivitätssensor (4) umfasst, der zur Erfassung mindestens einer lokalen Permittivität eingerichtet ist.

## Claims

1. Method for tuning (8) optical measurements on continuously stirred reactors (1), reactor contents (2) of the reactor (1) having at least one optically detectable measured variable, at least one optical measuring arrangement (3) being able to take, in particular taking, optical measurements of at least one of the optically detectable measured variables, at least one optical measurement being able to be tuned, in particular being tuned, to the form, distribution or movement state of at least one phase (2A, 2B) of the reactor contents (2), the reactor contents (2) being stirred causing local changes in the permittivity within the reactor (1), **characterized in that** the local permittivity is detected, in particular on the basis of a permittivity signal (6), at at least one location having a known distance from the at least one optical measuring arrangement (3) to be tuned, and **in that** the detected permittivity signal (6) from at least one location having a known distance from the at least one optical measuring arrangement (3) to be tuned is used to tune at least one optical measurement to the form, distribution or movement state of the reactor contents (2).

2. Method according to the preceding claim, **characterized in that** the change in at least one local permittivity or some other values derived therefrom are used to tune at least one optical measurement to the form, distribution or movement state of at least one phase (2A, 2B) of the reactor contents (2).

3. Method according to either of the preceding claims, **characterized in that** the tuning (8) of at least one optical measuring arrangement (3) is carried out as the activation and deactivation of the optical measuring arrangement (3) according to the form, distribution or movement state of at least one phase (2A, 2B) of the reactor contents (2) in a field of view of the optical measuring arrangement (3).

4. Method according to any of the preceding claims, **characterized in that** the tuning (8) of at least one optical measuring arrangement (3) is carried out as the correction or normalization of the optical measuring signal from the optical measuring arrangement (3) according to the form, distribution or movement state of at least one phase of the reactor contents (2) in a field of view of the optical measuring arrangement (3).

5. Method according to any of the preceding claims, **characterized in that** the form, distribution or movement state of the reactor contents (2) is detected by means of a plurality of permittivity sensors (4.1, 4.2, 4.3).

6. Method according to any of the preceding claims, **characterized in that** at least one local permittivity is detected by means of at least one electric field (5) which interacts locally with the reactor contents (2) and at least one permittivity sensor (4).

7. Method according to any of the preceding claims, **characterized in that** at least one local permittivity is detected by means of a capacitive measuring method, in particular using a capacitive permittivity sensor (4).

8. Device for carrying out the method according to any of the preceding claims, **characterized in that** the device comprises at least one permittivity sensor (4) at at least one location having a known distance from the at least one optical measuring arrangement (3) to be tuned, which sensor is designed to detect changes in the local permittivity, and **in that** the device comprises at least one control unit (7) which is designed to tune the optical measurement to the form, distribution or movement state of the reactor contents or at least one of its phases, on the basis of at least one permittivity signal (6) from at least one permittivity sensor (4).

9. Device according to the preceding claim, **characterized in that** the control unit (7) is designed to control a method according to any of claims 1 to 7.

10. Device according to either claim 8 or claim 9, **characterized in that** the device comprises at least one capacitive permittivity sensor (4) designed to detect at least one local permittivity.

## Revendications

1. Procédé de coordination (8) de mesures optiques dans des réacteurs (1) continuellement mélangés, dans lequel un contenu (2) du réacteur (1) présente au moins une grandeur de mesure pouvant être détectée par voie optique, dans lequel au moins un dispositif de mesure optique (3) peut effectuer, effectue notamment des mesures optiques d'au moins l'une des grandeurs de mesure pouvant être détectées par voie optique, dans lequel au moins une mesure optique doit être coordonnée, est notamment coordonnée avec la forme, la distribution ou l'état de mouvement d'au moins une phase (2A, 2B) du contenu du réacteur (2), dans lequel le mélange du contenu du réacteur (2) induit des modifications locales de la permittivité à l'intérieur du réacteur (1), **caractérisé en ce qu'**au niveau d'au moins un emplacement se trouvant à une distance connue de l'au moins un dispositif de mesure optique (3) à coordonner, la permittivité locale est détectée, en particulier au moyen d'un signal de permittivité (6), et **en ce que** le signal de permittivité (6) détecté d'au moins un emplacement se trouvant à une distance connue de l'au moins un dispositif de mesure optique (3) à coordonner permet de coordonner au moins une mesure optique avec la forme, la distribution ou l'état de mouvement du contenu du réacteur (2).

2. Procédé selon la revendication précédente, **caractérisé en ce que** la modification d'au moins une permittivité locale ou d'autres valeurs dérivées de celle-ci permettent de coordonner au moins une mesure optique avec la forme, la distribution ou l'état de mouvement d'au moins une phase (2A, 2B) du contenu du réacteur (2).

3. Procédé selon l'une des revendications précédentes, **caractérisé en ce que** la coordination (8) d'au moins un dispositif de mesure optique (3) se déroule comme une activation et une désactivation du dispositif de mesure optique (3) en fonction de la forme, de la distribution ou de l'état de mouvement d'au moins une phase (2A, 2B) du contenu du réacteur (2) dans un champ de vision du dispositif de mesure optique (3).

4. Procédé selon l'une des revendications précédentes, **caractérisé en ce que** la coordination (8) d'au moins un dispositif de mesure optique (3) se déroule comme une correction ou une normalisation du signal de mesure optique du dispositif de mesure optique (3) en fonction de la forme, de la distribution ou de l'état de mouvement d'au moins une phase du contenu du réacteur (2) dans un champ de vision du dispositif de mesure optique (3).

5. Procédé selon l'une des revendications précédentes, **caractérisé en ce que** la détection de la forme, de la distribution ou de l'état de mouvement du contenu du réacteur (2) est effectuée par une pluralité de capteurs de permittivité (4.1, 4.2, 4.3).

6. Procédé selon l'une des revendications précédentes, **caractérisé en ce que** la détection d'au moins une permittivité locale s'effectue par l'intermédiaire d'au moins un champ électrique (5), lequel interagit localement avec le contenu du réacteur (2) et au moins un capteur de permittivité (4).

7. Procédé selon l'une des revendications précédentes, **caractérisé en ce que** la détection d'au moins une permittivité locale s'effectue par l'intermédiaire d'un procédé de mesure capacitif, notamment à l'aide d'un capteur de permittivité capacitif (4).

8. Dispositif de mise en oeuvre du procédé selon l'une des revendications précédentes, **caractérisé en ce que** le dispositif comprend au moins un capteur de permittivité (4) au niveau d'au moins un emplacement se trouvant à une distance connue de l'au moins un dispositif de mesure optique (3) à coordonner, lequel est conçu pour détecter des modifications de la permittivité locale, et **en ce que** le dispositif comprend au moins une unité de commande (7), laquelle est conçue, sur la base d'au moins un signal de permittivité (6) d'au moins un capteur de permittivité (4), pour réaliser la coordination de la mesure optique avec la forme, la distribution ou l'état de mouvement du contenu du réacteur ou d'au moins l'une de ses phases.

9. Dispositif selon la revendication précédente, **caractérisé en ce que** l'unité de commande (7) est conçue pour commander un procédé selon l'une des revendications 1 à 7.

10. Dispositif selon l'une des revendications 8 à 9, **caractérisé en ce que** le dispositif comprend au moins un capteur de permittivité capacitif (4) conçu pour détecter au moins une permittivité locale.
